# EUROPÄISCHE PATENTSCHRIFT

(11) **EP 1 654 976 B1**
(45) Veröffentlichungstag und Bekanntmachung des Hinweises auf die Patenterteilung: **08.06.2016**
(21) Anmeldenummer: 05022928.5
(22) Anmeldetag: 20.10.2005
(51) Int. Cl.: A61B 1/01, A61M 25/01, A61B 1/012

(54) **Endoskop mit alternierendem Vortrieb**
Endoscope with alternating driving
Endoscope à progression alternée

(30) Priorität: 26.10.2004 DE 102004052036
(43) Veröffentlichungstag der Anmeldung: 10.05.2006
(73) Patentinhaber: invendo medical GmbH, 69469 Weinheim (DE)
(72) Erfinder: Bob, Konstantin, Dr., 69469 Weinheim (DE); Pauker, Fritz, 86438 Kissing (DE); Viebach, Thomas, 82282 Pischertshofen (DE)
(74) Vertreter: Winter, Brandl, Fürniss, Hübner, Röss, Kaiser, Polte - Partnerschaft mbB

(56) Entgegenhaltungen:
- EP-A- 1 582 139
- DE-A1- 3 630 660
- US-A- 4 577 621
- US-A- 4 815 450
- US-A- 5 112 310
- US-A- 6 007 482
- US-A1- 2003 114 803
- US-B1- 6 464 665

## Beschreibung

Die Erfindung bezieht sich auf ein Endoskop mit alternierendem Vortrieb zum Bewegen eines Endoskopschafts längs eines kanalartigen Hohlraums gemäß dem Oberbegriff des Patentanspruchs 1.

Endoskope sind zu einem wichtigen Hilfsmittel in der Technik und vor allem in der Medizin geworden, um kanalartige Hohlräume zu inspizieren, die ansonsten nur mit erheblichen Eingriffen zugänglich sind. Bevorzugt werden Endoskope zur Untersuchung der Speiseröhre, des Magens, des Zwölffingerdarms vom Magen aus, des Darms vom Anus aus, der Harnröhre, der Blase und des Harnleiters verwendet.

Derartige Endoskope sind an ihrem Vorderende mit einer Beleuchtungseinrichtung und mit einer Optik zur visuellen Erfassung des davorliegenden Bereichs des Hohlraums ausgerüstet. Die am Vorderende des Endoskops erfasste, optische Information wird normalerweise entweder mittels einer Faseroptik durch das Endoskop nach hinten zu seinem Bedienungsende übertragen oder wird mittels eines optischen Sensorchips am Vorderende erfasst, durch eine elektrische Leitung durch das Endoskop nach hinten geleitet und auf einem Bildschirm sichtbar gemacht. Darüber hinaus ist auch eine Informationsübertragung per Funk vom Vorderende zum Bedienungsende denkbar.

Ferner weisen Endoskope in der Regel einen sogenannten Arbeitskanal auf, durch den diverse Arbeitsinstrumente eingeführt und bedient werden können. Beispielsweise werden kleine Zangen zur Entnahmen von Gewebeproben, Biopsienadeln, beheizbare Schneiddrähte, kleine Scheren, Koagulationselektroden oder dergleichen eingeführt, um ggf. operative Maßnahmen an beschädigtem Gewebe durchzuführen. Schließlich sind in der Regel ein Fluidkanal für Spülflüssigkeit und Bedienungsdrähte oder Fluidleitungen zum Abwinkeln des Endoskopvorderendes in mehrere Richtungen vorhanden. Diese Bedienungsdrähte oder Fluidleitungen werden wiederum innerhalb des Endoskopschafts zu dessen vorderem bzw. distalem Ende geführt.

Zum Einführen und Vorwärtsbewegen des Endoskopschafts im zu inspizierenden kanalartigen Hohlraum offenbart die DE 42 44 990 eine entsprechende Vorrichtung. Bei dieser Vorrichtung wird bei der Vorwärtsbewegung des Endoskopschafts in einen Hohlraum ein Stülpschlauch mit dem Endoskopschaft mitbewegt und bei erreichen eines Distalendes vorwährend nach Außen gestülpt, so dass der nach Außen gestülpte Abschnitt des Stülpschlauchs gegenüber der Hohlraumwand ruht. Der Vorteil dieses ruhenden Stülpschlauchabschnitts besteht darin, dass Beschädigungen der Hohlraumwand verringert werden. Außerdem wurde bei dieser Vorrichtung davon ausgegangen, dass bei medizintechnischer Anwendung die Schmerzen für den Patenten aufgrund der Vorwärtsbewegung mit relativ zur Darmwand ruhendem Stülpschlauch entscheidend verringert werden können.

Neuere Untersuchungen zeigten jedoch, dass nicht die Relativbewegung zwischen dem eingeführten Endoskopschaft und der Darmwand die Hauptursache für die Schmerzen bei einer Darmspiegelung darstellen, sondern dass die Schmerzen vor allem durch Drücken des Distalendes des Endoskopschafts gegen die Krümmungsaußenwand von Darmbiegungen entstehen. (Die Begriffe Krümmungsaußenwand und -innenwand bezeichnen in diesem Zusammenhang jeweils die bezüglich eines fiktiven Mittelpunktes einer Hohlraumbiegung jeweils weiter außen- bzw. innenliegende Hohlraumwandung.)

Als weiteren relevanten Stand der Technik seien noch die folgenden Druckschriften genannt:
Die US 4,815,450 offenbart ein Endoskop mit einer schlauchförmigen Einführhilfe, wobei durch Vakuumisieren, bzw. durch Kühlen und Erwärmen des Endoskopschafts und des Schlauchs die jeweiligen Materialsteifigkeiten temporär veränderbar sind. Auf diese Weise können sich der Endoskopschaft und die Einführhilfe bei alternierendem Vorschub gegenseitig abstützen. Jedoch ist die Stützkraft insbesondere bei großer Vorschubstrecke nicht ausreichend.

Die DE 36 30 660 A1 offenbart ein Coloskop bestehend aus zwei Hauptelementen, nämlich einem Gleit- und einem Hüllrohr, die jeweils an ihrem distalen Ende mit aufweitbaren Ballons versehen sind. Diese Ballons werden abwechselnd aufgebläht und stützen sich dabei an der Darmwand ab. Sie bilden somit eine Art Widerlager an denen Zugkräfte in die Darmwand eingeleitet werden, die beim Hineinziehen des jeweils gerade nicht abgestützten Hauptelements entstehen. In sofern entspricht der in der DE 36 30 660 A1 offenbarte Vortrieb einer Art "inchworm"-Bewegung, bei dem sich abwechselnd ein hinteres und ein vorderes Lager an einem fixen Gegenstand abstützt. Hierbei werden sämtliche Vorschubkräfte in die Darmwand eingeleitet, was zu Schmerzen beim Patienten führt.

Die US 6,007,482 sowie die US 4,557,621 offenbaren jeweils ebenfalls ein Endoskop, das nach dem "inchworm"-Bewegungsprinzip in einen zu untersuchenden Hohlraum vorwärts bewegt wird. Somit entsprecht die US 6,007,482 und die US 4,557,621 im Grunde der DE 36 30 660 A1 mit allen damit verbundenen Nachteilen.

Daher ist es eine Aufgabe der Erfindung, ein Endoskop mit einem Vortriebsystem einfachen Aufbaus zu realisieren, mit dem weniger Druck gegen die Krümmungsaußenwand ausgeübt wird.

### ZUSAMMENFASSUNG DER ERFINDUNG

Diese Aufgabe wird durch ein Endoskop mit alternierendem Vortriebsystem mit den Merkmalen des Anspruchs 1 gelöst. Die Erfindung beruht dabei auf folgender Grundüberlegung.

Endoskopschäfte müssen prinzipiell zwei zueinander gegensätzliche Bedingungen erfüllen. Zum Einen müssen sie in einen zu untersuchenden Hohlraum geschoben werden, was eine bestimmte Steifigkeit des Schafts voraussetzt, um nicht auszuknicken. Zum Anderen müssen sie flexibel genug sein, Krümmungen folgen zu können. Letztere Bedingung ist insbesondere bei Untersuchungen von Darmwandungen wichtig, da eine zu geringe Flexibilität dazu führt, dass ein Endoskopschaft an einer zu passierenden Darmkrümmung die Darmwand ausbeult, was, wie vorstehend ausgeführt wurde, zu erheblichen Schmerzen führt.

Gemäß einem Aspekt der Erfindung wird ein Endoskopschaft mit Hilfe einer Hilfseinrichtung vorzugsweise in Form eines Schlauchs oder Schlaucheinrichtung in einem kanalartigen Hohlraum vorangetrieben wird, welcher den Schaft außenwandig umgibt. Entscheidend dabei ist, dass dieser Schlauch/Schlaucheinrichtung zumindest temporär steifer ist oder gemacht werden kann, wie der vorzugsweise hochflexible Endoskopschaft. Durch diese Ausgestaltung wird ein Ausknicken des Endoskopschafts durch den diesen umgebenden und stützenden Schlauchs/Schlaucheinrichtung verhindert, gleichzeitig kann eine immer noch ausreichend hohe Flexibilität beibehalten werden.

Der Kern der Erfindung zur Umsetzung des vorstehenden prinzipiellen Erfindungsgedankens besteht demzufolge darin, den außenseitigen Schlauch/Schlauchhilfseinrichtung relativ bewegbar zum darin liegenden Endoskopschaft zu gestalten, wobei der Endoskopschaft als das erste Element und die Hilfseinrichtung als das zweite Element abwechselnd bezüglich eines zu inspizierenden Hohlraums ruhend sind, d.h. sich nicht nach vorn bewegt, und gleichzeitig das gerade nicht ruhende Element unter Führung entlang des gerade ruhenden Elements in den Hohlraum vorantreibbar ist.

Erfindungsgemäß ist es vorgesehen, dass eines der beiden Elemente, nämlich der den Endoskopschaft umgebende Schlauch/Schlaucheinrichtung, nur temporär eine größere Steifigkeit erhält als jene des Endoskopschafts. Konkreter ausgedrückt ist die Grundsteifigkeit des Schlauchs geringer als die des Endoskopschafts. In dieser Situation dient der Endoskopschaft als Führungselement, d.h. ist der Endoskopschaft in der Lage den relativ zu diesem sich bewegenden Schlauch auch durch Krümmungen zu führen. Darüber hinaus weist der Schlauch eine Vorrichtung auf, mittels der der flexible Schlauch ausgesteift werden kann, insbesondere auf eine solche Steifigkeit, die größer ist als die des Endoskopschafts.

In dieser Situation dient der Schlauch als Führungselement, d.h. ist der Schlauch in der Lage, den relativ zu diesem sich bewegenden Schaft durch Krümmungen zu führen. Dabei stehen dem Fachmann eine Vielzahl von derartigen Vorrichtungen zur wahlweisen (temporären) (alternierenden) Aussteifung des Schlauchs und/oder des Schafts zur Verfügung. Erfindungsgemäß, ist der Schlauch mit Fluidkissen ausgekleidet oder bedeckt, deren Inhalt (Volumen) wahlweise eingespannt und wieder freigegeben werden kann.

Weitere vorteilhafte Ausgestaltungen der Erfindung sind Gegenstand der übrigen Patentansprüche.

### KURZBESCHREIBUNG DER ZEICHNUNGEN

Die Erfindung wird nachstehend anhand eines bevorzugten Ausführungsbeispiels bezugnehmend auf die beigefügten Zeichnungen genauer beschrieben, dabei ist in den Zeichnungen folgendes schematisch dargestellt:
Fig. 1 ist eine räumliche Darstellung eines bevorzugten Ausführungsbeispiels des Endoskops der vorliegenden Erfindung;
Fig. 2 stellt Antriebseinrichtungen zum Vorwärtsbewegen des Endoskops gemäß des bevorzugten Ausführungsbeispiels dar;
Die Fig. 3A bis 3C sind Vorderansichten des Endoskops, welche die Vorwärtsbewegung des Endoskops gemäß des bevorzugten Ausführungsbeispiels veranschaulichen;

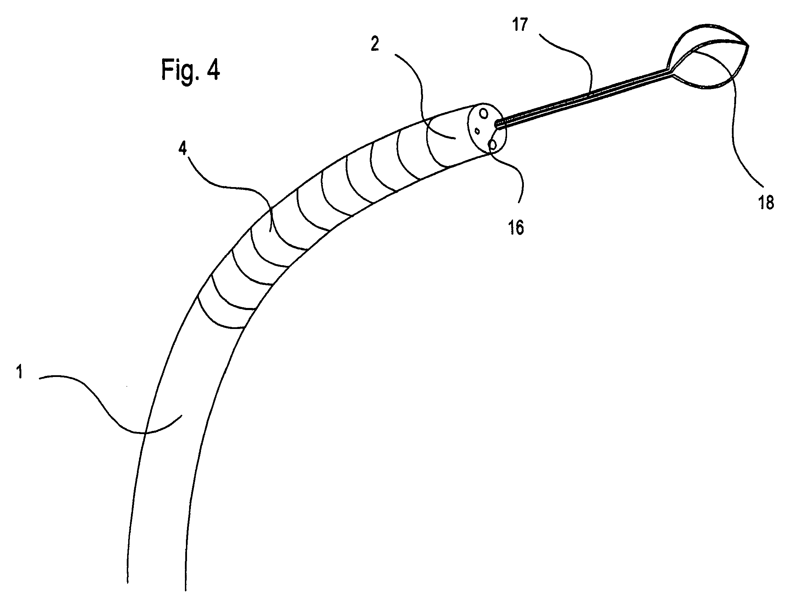

Die Fig. 4A bis 4C sind Vorderansichten des Endoskops, welche die Vorwärtsbewegung des Endoskops gemäß einer Modifikation des bevorzugten Ausführungsbeispiels veranschaulichen und

### DETAILLIERTE BESCHREIBUNG DER BEVORZUGTEN AUSFÜHRUNGSBEISPIELE

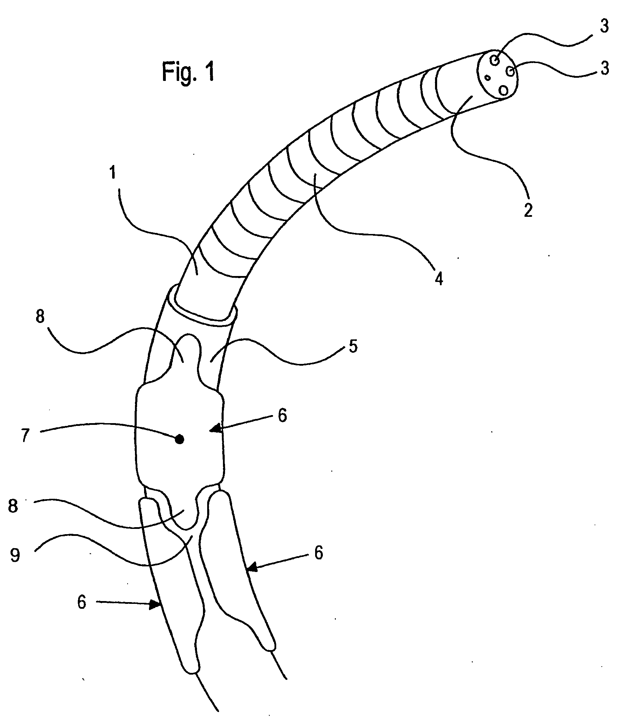

Anhand der Figuren 1 bis 3C wird das Endoskop mit alternierendem Vortrieb gemäß einem bevorzugten Ausführungsbeispiel näher beschrieben. Gemäß diesem Ausführungsbeispiel besteht das erfindungsgemäße Endoskop aus einem flexiblen Endoskopschaft 1, an dessen Distalende ein Endoskopkopf 2 angefügt ist und an dessen anderem Ende (Bedienungsende) Bedienungs- und ggf. Informationsauswertungsgeräte (nicht dargestellt) angeschlossen sind. Dieser Endoskopkopf 2 kann mit verschiedenen Einrichtungen 3, wie beispielsweise einer Optik, einer Beleuchtungseinrichtung, einem Arbeitskanal für verschiedene Werkzeuge usw. ausgebildet werden.

Der Endoskopkopf 2 ist vorzugsweise über einen beweglichen distalen Endabschnitt 4 (nachfolgend als "Deflecting" bezeichnet) mit dem flexiblen Endoskopschaft 1 verbunden. Das Deflecting 4 kann ansprechend auf Anweisungen vom Bedienungsende (nicht dargestellt) des Endoskops in verschiedenen Krümmungsradien vorzugsweise in mehrere Richtungen gekrümmt werden. Verwendung findet hierbei ein herkömmliches Deflecting, wie beispielsweise in den Anmeldungen DE 102 09 986 A1 oder DE 100 10 932 A1 beschrieben. Bezüglich der genauen Funktionsweise und Ausführung dieses Deflectings wird auf diese Anmeldungen verwiesen. An dieser Stelle soll lediglich festgehalten werden, dass wie bereits erwähnt, das Deflecting durch Steuersignale bzw. -drücke vom Bedienungsende gekrümmt werden kann und somit auch wahlweise in dieser gekrümmten Stellung oder in einer nicht gekrümmten Stellung versteift werden kann.

Die Leitungen (nicht dargestellt) zum Ansteuern des Deflectings 4 und der Einrichtungen 3 sowie eventuell vorhandene Arbeitskanäle zum Bedienen und Einführen von am Endoskopkopf 2 verwendeten Werkzeugen verlaufen innerhalb des Deflectings 4 und des Endoskopschafts 1 zum Bedienungsende nach hinten.

Der Endoskopschaft 1 wird bevorzugterweise bis zu der Stelle, an der der Endoskopschaft 1 mit dem Deflecting 4 verbunden ist von einem vorzugsweise einwandigen Schlauch 5 umgeben, so dass am Vorderende (distales Ende) des Schlauchs 5 das Deflecting 4 mit dem damit verbundenen Endoskopkopf 2 herausragen. Der Schlauch besteht in diesem Ausführungsbeispiel aus EPTFE-Material, dessen Wandstärke 2 - 3mm beträgt. Zur Verstärkung kann eine Spiralfeder in das schlauchförmige EPTFE-Material derart eingebracht sein, dass das EPTFE-Schlauchmaterial konisch zur Spiralfeder verläuft und der Federdraht (nicht notwendiger Weise aus Federstahl gefertigt) zwischen Schlauchinnenwand und Schlauchaußenwand komplett im EPTFE-Material eingebettet wird. Außerdem ist es auch denkbar, die Spiralfeder konisch zum EPTFE-Schlauchmaterial anzuordnen, wobei die Spiralfeder vom EPTFE-Schlauchmaterial eingehüllt und mit diesem verbunden ist, so dass die Spiralfeder nach Innen freigelegt ist. Darüber hinaus ist die Spiralfeder nicht unbedingt erforderlich und es ist auch eine Ausführung des Schlauchs 5 ohne Spiralfeder denkbar. Das Schlauchmaterial ist ebenfalls nicht auf das genannte EPTFE-Material begrenzt und kann aus anderen Materialien wie beispielsweise Silikon ausgebildet sein.

Der Schlauch 5 ist in diesem Ausführungsbeispiel am Außenumfang mit Fluidkissen 6 versehen. Diese Fluidkissen 6 sind vorzugsweise in einer länglichen Gestalt ausgebildet und an deren Mittelabschnitten 7 verbreitert. Somit weisen die Fluidkissen 6 an ihrem Vorder- und Hinterende einen schmalen zungenförmigen Endabschnitt 8 auf.

Im vorliegenden Ausführungsbeispiel sind die Fluidkissen 6 jeweils in Gruppen, bestehend aus drei Fluidkissen 6 angeordnet. Die Fluidkissen 6 dieser Dreier-Gruppe werden in der Längsrichtung parallel zueinander um den Außenumfang des Schlauchs 5 ausgerichtet, so dass sich die Seitenflächen der Fluidkissen 6 an ihrem Mittelabschnitt 7 berühren oder einen kleinen Zwischenraum ausbilden. Das heißt, diese Dreier-Gruppe an Fluidkissen ist derart um den Außenumfang des Schlauchs verteilt, dass die Mittelsenkrechten der Fluidkissen 6 sich mit der Mittelachse des Endoskopschafts 1 schneiden und zur Mittelsenkrechten des jeweils benachbarten Fluidkissens 6 einen Winkel von 120° bilden. Bei dieser Gruppe ragen somit die zungenförmigen Endabschnitte 8 im Abstand von 120° (in einer Ebene senkrecht zur Schlauch-Mittelachse) um den Außenumfang des Schlauchs 5 verteilt nach vorn und hinten. Da die Endabschnitte 8 schmäler als die Mittelabschnitte 7 sind, ist ein Endabschnitt zum jeweils benachbarten Endabschnitt weiter beabstandet als die jeweiligen Mittelabschnitte 7. Dadurch werden jeweils zwischen zwei benachbarten Endabschnitten 8 Ausbuchtungen 9 ausgebildet.

Benachbart zur obigen Dreier-Gruppe an Fluidkissen 6 ist vor und hinter dieser eine weitere Dreier-Gruppe an Fluidkissen 6 derart angeordnet, dass die Endabschnitte 8 der letzteren Dreier-Gruppe an Fluidkissen 6 in die Ausbuchtungen der ersteren Dreier-Gruppe an Fluidkissen 6 eingefügt sind. Das heißt in Längsrichtung benachbarte Fluidkissen-Gruppen sind in Umfangsrichtung in einer Ebene senkrecht zur Schlauch-Mittelachse um 60° versetzt.

Die Fluidkissen 6 sind entsprechend der soeben beschriebenen Art und Weise etwa über einen 50cm langen Endabschnitt des Schlauchs 5 ausgehend von dessen Vorderende angeordnet.

Die Fluidkissen 6 sind über Zuleitungen (nicht dargestellt), welche in der Wandung oder am Innen- oder Außenumfang des Schlauchs 5 nach hinten zum Bedienungsende verlaufen, mit Druck zu beaufschlagen.

Je nach Druckbeaufschlagung der Fluidkissen 6 kann die Flexibilität des Schlauchs 5 verändert werden. Außerdem legen sich die Luftkissen 6 an die Hohlraumwand an, so dass der Schlauch 5 gegenüber der Hohlraumwand feststehend ist. Eine Druckbeaufschlagung der Fluidkissen 6 bzw. ein Entspannen von inkompressiblem Fluid in den Fluidkissen 6 ermöglicht somit, den Schlauch 5 wahlweise mit einer Flexibilität auszustatten, die kleiner oder größer als die Flexibilität des Endoskopschafts 1 ist sowie gleichzeitig den Schlauch 5 gegenüber der Hohlraumwand in einen feststehenden oder bewegbaren Zustand zu versetzen.

Dadurch dass sich die Luftkissen an den zungenförmigen Endabschnitten 8 überlappen kann sich der Schlauch 5 auf dem ganzen Umfang der Hohlraumwand abstützen, ohne seine Biegsamkeit zu verlieren.

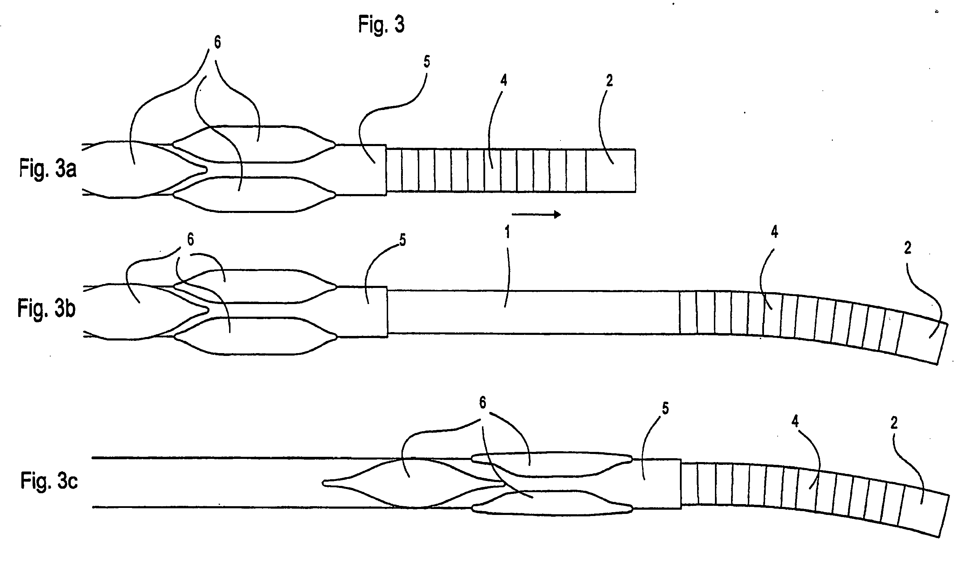

Fig. 3 zeigt das Endoskop in einem Zustand, bei dem es bereits ein Stück weit in den Hohlraum hineinbewegt worden ist. Bezugsnummer 10 kennzeichnet eine erste Antriebseinrichtung 10 zur Vorwärtsbewegung des Schlauchs 5. Die Antriebseinrichtung 10 besteht aus einem Paar Antriebsrädern 11 die mit ihrem rinnenförmigen Außenumfang von zwei Seiten her mit dem Schlauch 5 in reibschlüssigem Eingriff sind. Diese Antriebsräder 11 werden von einem nicht eingezeichneten Elektromotor angetrieben. Der Schlauch 5 ist mit seinem hinteren Ende an einem plattenartigen Teil 12 befestigt. Zwischen der ersten Antriebseinrichtung 10 und der Platte 12 ist der Endoskopschaft 1 mit dem umgebenden Schlauch 5 zu einer ausgebuchteten Form 15 gelegt. Im Bereich der Platte 12 könnte Schmiermittel in den Raum zwischen dem Endoskopschaft 1 und dem Schlauch 5 eingepresst werden.

Auf der dem Schlauch 5 abgewandten Seite der Platte 12 ist vorzugsweise ein Paar Antriebsräder 13 vorgesehen, die mit ihrem rinnenförmigen Außenumfang von zwei Seiten her mit dem Endoskopschaft 1 in reibschlüssigem Eingriff sind. Diese Antriebsräder 13 bilden zusammen mit einem nicht eingezeichneten Elektromotor und der mit den Antriebsrädern 13 verbundenen Platte 12 eine zweite Antriebseinrichtung 14.

Die erste Antriebseinrichtung 10 ist mit der zweiten Antriebseinrichtung 14 fest verbunden, so dass sich diese Antriebseinrichtungen nicht relativ zueinander bewegen können. Außerdem sind diese Antriebseinrichtungen 10, 14 möglichst so bezüglich des zu untersuchenden Hohlraums anzubringen, dass sie sich nicht relativ zum Hohlraum bewegen.

Die Oberfläche der Antriebsräder 11 an ihrem rinnenförmigen Außenumfang ist derart gestaltet, dass eine große Reibung zwischen den Antriebsrädern 11 und dem Schlauch 5 besteht. Zwischen dem Schlauch 5 und dem Endoskopschaft 1 ist entweder Schmiermittel eingebracht und/oder der Endoskopschaft 1 ist derart beschichtet (z.B. Teflon), dass zwischen Schlauch 5 und Endoskopschaft 1 eine sehr gering Reibung auftritt. Somit ist es möglich, dass der Schlauch 5 reibschlüssig mittels der Antriebsräder 11 angetrieben wird, während der Endoskopschaft 1 mittels der Antriebsräder 13 in die entgegengesetzte Richtung bewegt wird oder stillsteht.

### (Funktionsweise des bevorzugten Ausführungsbeispiels)

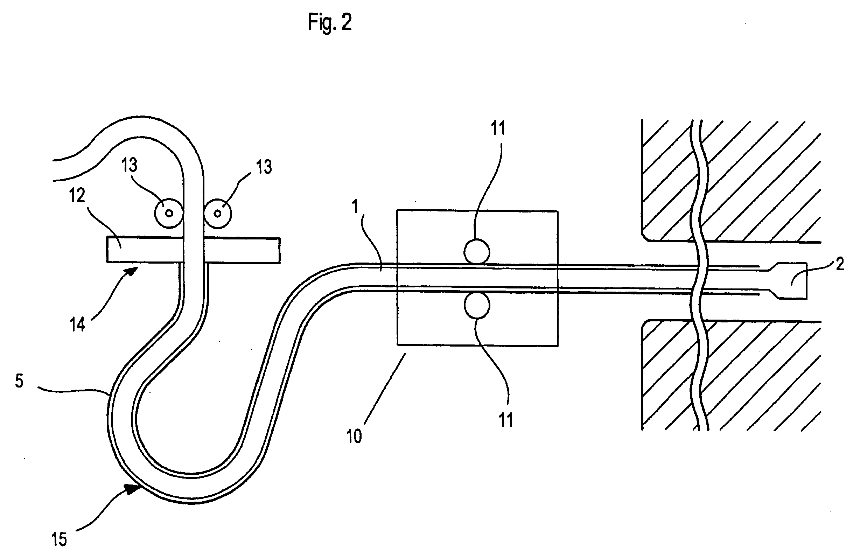

Die Funktionsweise des bevorzugten Ausführungsbeispiels wird unter Bezugnahme auf Figur 2 und 3 beschrieben.

In Figur 3a befindet sich der Endoskopschaft 1 und der Schlauch 5 an einer bestimmten Stelle eines zu inspizierenden Hohlraums. Die Fluidkissen 6 werden über die Pumpe (nicht dargestellt) mit Fluid gefüllt und legen sich somit an die Wand des Hohlraums an. Durch den Druck der Fluidkissen 6 gegen die Hohlraumwand, ist der Schlauch 5 bezüglich der Hohlraumwand feststehend. Zusätzlich oder alternativ wird der Schlauch 5 durch die Antriebseinrichtung 10 festgehalten. Außerdem wird die Fluidzufuhr derart gesteuert, dass der Schlauch 5 bei abgeschlossener Fluidkissenbefüllung eine geringere Flexibilität (höhere Steifigkeit) aufweist als der Endoskopschaft 1.

Von diesem Zustand ausgehend wird der Endoskopschaft 1 mittels der zweiten Antriebseinheit 14 vorwärtsbewegt. Die erste Antriebseinheit 10 steht dabei still. Dieser Vorgang ist in Figur 3b schematisch dargestellt. Beim Vorwärtsbewegen des Endoskopschafts 1 dient der Schlauch 5 als Stützkanal für die Bewegung des Endoskopschafts 1. Diese Stützwirkung wird durch die geringere Flexibilität des Schlauchs 5 unterstützt.

Sollte es während dieser Vorwärtsbewegung des Endoskopschafts 1 erforderlich sein, den vorderen Teil des Endoskopschafts 1 entlang einer Hohlraumbiegung zu führen wird folgendermaßen verfahren. Zunächst kann die Hohlraumbiegung am Endoskopkopf 2 durch Sensoren oder durch die im Endoskopkopf installierte Optik erkannt werden. Wenn der Endoskopkopf 2 entlang der Hohlraumbiegung vorwärts bewegt wird, kann die Krümmung des Deflectings 4 eingestellt und dem Krümmungsradius der Hohlraumbiegung angepasst werden. Somit wird kein oder nur wenig Druck auf die Krümmungsaußenwand des Hohlraums ausgeübt. Wenn der Endoskopkopf 2 ein Ende der Hohlraumbiegung erreicht hat, wird beim weiteren Vorwärtsbewegen des Endoskopkopfs 2 das Deflecting 4 wieder gerade ausgerichtet.

Wenn der Endoskopschaft 1 mittels der zweiten Antriebseinheit 14 eine bestimmte vorgegebene Strecke aus dem Schlauch 5 herausbewegt wurde (vorgeeilt ist), wird die zweite Antriebseinheit gestoppt, wodurch der Schaft 1 festgehalten wird.

Die weitere Vorwärtsbewegung ist in Figur 3c dargestellt. Demnach werden die Fluidkissen 6 entspannt, so dass der Schlauch 5 bezüglich der Hohlraumwand verschiebbar ist. Außerdem weist der Schlauch 5 mit entspannten Fluidkissen 6 eine größere Flexibilität als der Endoskopschaft 1 auf. Der Schlauch 5 wird in diesem Zustand durch die erste Antriebseinrichtung 10 vorwärtsbewegt. Während dieser Vorwärtsbewegung wird die ausgebuchtete Form 15 aus Endoskopschaft 1 und Schlauch 5 kleiner. Dadurch, dass die Antriebseinrichtung 14 den Endoskopschaft festhält, wird erreicht, dass der Endoskopschaft 1 bezüglich des zu inspizierenden Hohlraums stillsteht, während sich das Vorderende des Schlauch 5 in Richtung zum Vorderende des Endoskopschafts 1 bewegt. (Bezüglich der Vorwärtsbewegung des Schlauchs 5 sind einige Modifikationen möglich, die am Ende dieser Beschreibung beschrieben sind.)

Bei dieser Vorwärtsbewegung des Schlauchs 5 wirkt der bezüglich des Hohlraums ruhende Endoskopschaft 1 mit seiner geringeren Flexibilität (höhere Grundsteifigkeit verglichen mit der des Schlauchs 5) als Schiene für den Schlauch 5. Da sich der Endoskopschaft 1 weiter im Hohlraum befindet als der Schlauch 5, hat der Endoskopschaft 1 zum Zeitpunkt, bei dem der Schlauch 5 in Bewegung gesetzt wird, bereits die Form des Hohlraums, den der Schlauch 5 in einem Zyklus durchfährt, mit allen eventuellen Biegungen angenommen. Wenn somit der Schlauch 5 der Form des Endoskopschafts 1 folgt, kann somit die Kraft, mit der der Schlauch 5 gegen die Krümmungsaußenwand drückt, verringert werden. Die ansonsten gegen die Krümmungsaußenwand drückende Kraft wird in der vorliegenden Erfindung vom in diesem Zustand steiferen Endoskopschaft 1 abgeleitet. Für die medizinische Anwendung bedeutet dies gleichzeitig, dass die vom Patienten wahrgenommenen Schmerzen nicht entstehen oder vermindert werden.

Der Schlauch 5 wird, wie in Figur 3c dargestellt, bevorzugterweise soweit vorwärts bewegt, bis das Vorderende des Schlauchs 5 die Verbindungsstelle zwischen Schlauch 5 und Deflecting 4 erreicht. Nach dem Durchlauf eines solchen Bewegungszyklus, wie er anhand der Figuren 3a bis 3c beschrieben wurde, wird die Vorwärtsbewegung des Endoskops mit einem erneuten Zyklus beginnend bei Figur 3a fortgesetzt. Das heißt, die Fluidkissen 6 werden wieder geflutet, so dass der Schlauch 5 relativ zur Hohlraumwand feststehend ist. Anschließend wird der Endoskopschaft 1 nach vorne bewegt usw.

Dieser alternierende Vortrieb von Endoskopschaft 1 und Schlauch 5 wird solange wiederholt, bis die gewünschte zu untersuchende Stelle im Hohlraum erreicht ist.

Das Rückwärtsbewegen des Endoskops kann durch Umkehrung des Ablaufs für die Vorwärtsbewegung erfolgen. Eine andere Möglichkeit wäre, die Fluidkissen zu entspannen und das Deflecting 4 so zu lösen, dass es frei beweglich ist. Anschließend könnte der Endoskopschaft zusammen mit dem Schlauch 5 aus dem Hohlraum herausgezogen werden.

### (Weitere Variationsmöglichkeiten)

Schließlich sei darf hingewiesen, dass die hier vorliegende Beschreibung und die beigefügten Figuren nur beispielhafter Natur sind und keinesfalls dazu dienen sollen, die Erfindung auf die hier vorgestellten Ausführungen zu begrenzen. Die Erfindung lässt eine Vielzahl von Anwendungsmöglichkeiten und Modifikationen zu ohne den Kern der Erfindung zu verlassen.

### Einige Modifikationen werden im Folgenden genannt:

Grundsätzlich kann das vorangehend beschriebene Prinzip des alternierenden Vortriebs des Endoskops in jedem beliebigen Stadium des Vorwärtsbewegens des Endoskops einsetzen bzw. wieder aussetzen. Das heißt, der Endoskopschaft und die Hilfseinrichtung können zuerst in den kanalartigen Hohlraum hineinbewegt werden und der alternierende Vortrieb wird erst an einer bestimmten, schwierig zu passierenden Stelle des Hohlraums eingesetzt. Nach dem Passieren dieser Stelle (z.B. starke Hohlraumbiegungen) ist auch ein Abschalten sowie ein erneutes Anschalten des alternierenden Vortriebs beim weiteren Vorwärtsbewegen möglich.

Im bevorzugten Ausführungsbeispiel wurde die Form der Fluidkissen spezifiziert. Jedoch ist jede beliebige andere Formgebung der Fluidkissen denkbar. Beispielsweise könnten die Fluidkissen durch einen Ring ausgebildet werden, der den Schlauch 5 in einer Ebene senkrecht zur Längsachse des Schlauchs umgibt. Dabei müsste der Ring innen hohl und in mehrere Kammern unterteilt sein. Die einzelnen somit entstandenen Kammern würden dann der Funktion jeweils eines Fluidkissens 6 entsprechen und wären ähnlich der Fluidkissen über Zuleitungen mit Druck zu beaufschlagen und zu entspannen.

Darüber hinaus kann auch ganz auf Fluidkissen 6 verzichtet werden und die Flexibilitätsveränderung findet über kanalartige Hohlräume statt, die in Längsrichtung am Außenumfang, am Innenumfang oder in der Wandung des Schlauchs bzw. der Hilfseinrichtung verlaufen. Außerdem könnten diese kanalartigen Hohlräume spiralförmig am Außenumfang, am Innenumfang oder in der Wandung des Schlauchs bzw. der Hilfseinrichtung ausgebildet sein. Ebenso kann eine Flexibilitätsveränderung des Endoskopschafts 1 vorzugsweise mittels dieser kanalartigen Hohlräume, die in Längsrichtung oder spiralförmig entlang des Endoskopschafts 1 verlaufen, realisiert werden, indem die kanalartigen Hohlräume am Außenumfang, im radial äußeren Bereich oder in einem Arbeitskanal angeordnet werden. Zur Flexibilitätsveränderung der Hilfseinrichtung und/oder des Endoskopschafts wird dann das sich in den Hohlräumen befindliche Fluid bzw. Medium druckbeaufschlagt bzw. entspannt.

Die Länge des vorderen Endabschnittes des Schlauchs 5, über die Fluidkissen 6 angebracht werden, beträgt im bevorzugten Ausführungsbeispiel etwa 50cm. Jedoch ist offensichtlich, dass die Länge dieses vorderen Endabschnittes entsprechend verändert werden kann.

Desweiteren sind die Fluidkissen 6 im bevorzugten Ausführungsbeispiel am Außenumfang des Schlauchs 5 angebracht. Eine Einbettung der Fluidkissen in der Schlauchwandung ist jedoch ebenfalls möglich.

Alternativ zum Schlauch 5 könnte im bevorzugten Ausführungsbeispiel eine Spiralfeder verwendet werden. Diese Spiralfeder könnte mit am Außenumfang angeordneten Fluidkissen als Hilfseinrichtung verwendet werden.

Die Anordnung der Fluidkissen erfolgte im bevorzugten Ausführungsbeispiel in Gruppen zu jeweils drei Fluidkissen. Die Anzahl der Fluidkissen pro Gruppe kann jedoch auch erhöht werden.

Die im bevorzugten Ausführungsbeispiel beschriebene Flexibilitätsveränderung des Schlauchs 5 mittels der Druckbeaufschlagung bzw. Entspannung der Fluidkissen 6 kann auch in mehreren Stufen oder gar kontinuierlich ausgeführt werden. Das heißt die Druckbeaufschlagung der Fluidkissen 6 könnte so gesteuert werden, dass sie umso höher druckbeaufschlagt werden, je weiter der Endoskopkopf 2 bei ruhendem Schlauch 5 in einer Hohlraumbiegung vorwärtsbewegt wird, das heißt je mehr das Deflecting 4 während der Vorwärtsbewegung abgekrümmt wird.

Im bevorzugten Ausführungsbeispiel schließt das Vorderende (distales Ende) des Schlauchs 5 im eingefahrenen Zustand des Endoskopschafts 1 (Zustand aus Fig. 3a) mit dem Hinterende des Deflectings 4 ab. Jedoch ist die vorliegende Erfindung nicht darauf zu beschränken und das Vorderende des Schlauchs 5 kann im eingefahrenen Zustand des Endoskopschafts 1 auch weiter hinterhalb des Deflectings 4 abschließen.

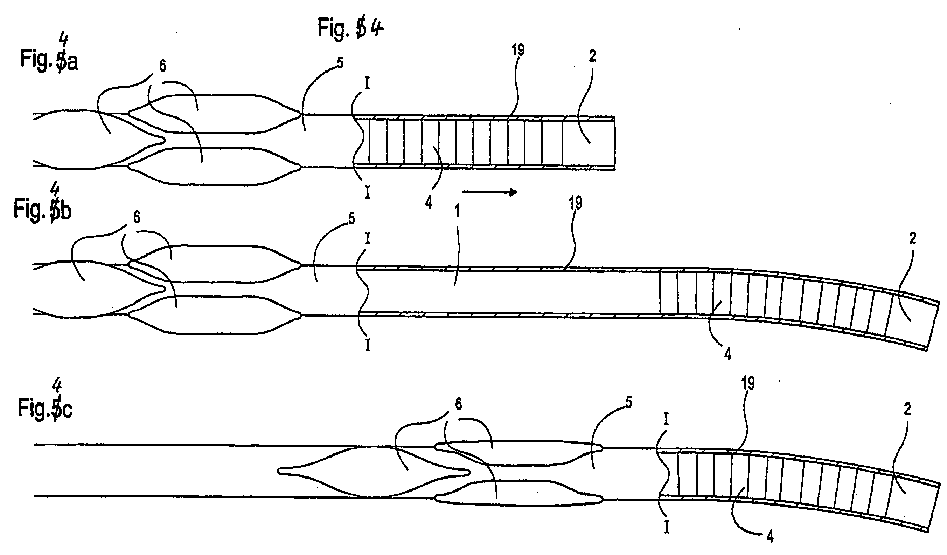

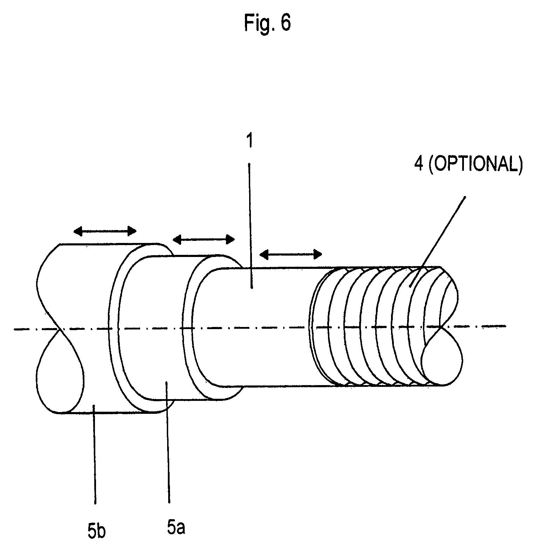

Da der Schlauch 5 bevorzugter Weise aus hoch dehnbarem (hoch flexiblem) EPTFE-Material hergestellt ist, kann der Schlauch stark ausgedehnt werden, ohne seinen Durchmesser bzw. seine Wandstärke zu verändern. Daher kann beispielsweise der Schlauch 5 im bevorzugten Ausführungsbeispiel bis zum Endoskopkopf 2 reichen und dort an diesem fluiddicht befestigt (z.B. durch Ankleben) sein. Dieser Zustand ist in Figur 5a dargestellt, dabei ist zu beachten, dass in den Figuren 5a bis 5c rechts der Linie I-I das Endoskop im Längsschnitt dargestellt ist. Im eingefahrenen Zustand des Endoskopschafts 5 (Zustand aus Figur 5a) sind hinterhalb des Deflectings 4 die Fluidkissen 6 angeordnet.

Wenn nun der Endoskopschaft 1 wie in Figur 5b dargestellt nach vorn bewegt wird während die Fluidkissen druckbeaufschlagt sind und somit bezüglich der Hohlraumwand ruhen, wird ein Dehnungsabschnitt 19 des Schlauchs 5 an dem keine Fluidkissen 6 angebracht sind, durch die Vorwärtsbewegung des Endoskopschafts 1 relativ zum Schlauch 5 ausgedehnt. Bei dieser Ausdehnung ist das Vorderende des Dehnungsabschnittes 19 am sich nach vorn bewegenden Endoskopkopf 2 fixiert und das Hinterende des Dehnungsabschnittes 19 ist durch die gegen die Hohlraumwandung drückenden Fluidkissen 6 gegenüber der Hohlraumwandung fixiert.

Wenn der Schlauch 5 mit entspannten Fluidkissen 6 nach vorne bewegt wird, wie in Figur 5c dargestellt, zieht sich der Dehnungsabschnitt 19 des Schlauchs 5 wieder zusammen.

Diese Modifikation ist als Zusatz zum ersten Aüsführungsbeispiel zu verstehen und hat den Vorteil, dass der Zwischenraum zwischen Endoskopschaft 1 und Schlauch 5 verschlossen ist, so dass weder Schmiermittel aus dem Zwischenraum austreten kann, noch Verunreinigungen in den Zwischenraum eintreten können.

Beim bevorzugten Ausführungsbeispiel wurde während des Vortriebs des Schlauchs 5 durch die erste Antriebseinheit 10 der Endoskopschaft 1 durch die zweite Antriebseinheit 14 mit gleicher Geschwindigkeit in die entgegengesetzte Richtung angetrieben.

Alternativ zum entgegengesetzten Antrieb des Endoskopschafts 1 durch die erste Antriebseinheit 14 könnte auch der Endoskopschaft 1 an dessen Vorderende mit Hilfe des Deflectings 4 bezüglich der Hohlraumwand fixiert werden und die erste Antriebseinheit 14 in Leerlauf geschaltet werden, so dass sich die Antriebsräder 13 frei drehen können. Somit würde der Endoskopschaft 1 ebenfalls gegenüber der Hohlraumwand ruhen während der Schlauch 5 nach vorn bewegt wird.

Eine weitere Alternative wäre, den Schlauch 5 nicht mit der Platte 12 zu verbinden. Dabei muss das Hinterende des Schlauchs 5 von der Platte 12 in dem Zustand, bei dem sich der Schlauch 5 bezüglich des Endoskopkopfs 2 in seiner vorderen Stellung befindet, um eine Strecke beabstandet sein, um die sich während eines Bewegungszyklus der Endoskopschaft 1 nach vorn bewegt. Beim Vortrieb des Endoskopschafts 1 wäre bei dieser Alternative das Hinterende des Schlauchs 5 nahe zur Platte 12 angeordnet und die Antriebsräder 11 der ersten Antriebseinheit 10 in Stillstand, so dass der Schlauch 5 bezüglich der Hohlraumwand ruht. Beim Vortrieb des Schlauchs 5 wäre allerdings die zweite Antriebseinheit 14 im Stillstand, so dass sich die Antriebsräder 13 nicht drehen. Die ausgebuchtete Form 15 würde bei dieser Vorwärtsbewegung des Schlauchs 5 nicht in ihrer Größe verändert werden und das Hinterende des Schlauchs 5 würde sich gleichzeitig mit dem Vortrieb in den Hohlraum auch weg von der Platte 12 bewegen.

Im bevorzugten Ausführungsbeispiel wurde die Flexibilitätsveränderung des Schlauchs 5 mittels der Fluidkissen 6 beschrieben. Zusätzlich dazu kann auch die Flexibilität des Endoskopschafts durch am Außenumfang des Endoskopschaft angeordnete oder im Außenumfang integrierte Fluidkissen variiert werden. Die Zuleitungen für diese Fluidkissen des Endoskopschafts müssten im Inneren des Endoskopschafts zum Bedienungsende nach hinten verlaufen. Der alternierende Vortrieb gemäß dieser Modifikation verläuft nach der gleichen Funktionsweise wie das erste Ausführungsbeispiel, wobei das temporär steifere Element aus Endoskopschaft und Schlauch das gleichzeitig flexiblere Element bei der Vorwärtsbewegung unterstützt.

## Patentansprüche

1. Endoskop mit alternierendem Vortrieb, mit einem Endoskopschaft (1) und einer Hilfseinrichtung (5), wobei das Endoskop durch abwechselndes Festhalten des Endoskopschafts (1) und der Hilfseinrichtung (5) bezüglich eines zu inspizierenden kanalartigen Hohlraums und gleichzeitiges Vorantreiben des gerade nicht festgehaltenen Elements unter Führung entlang des gerade festgehaltenen Elements in den Hohlraum vorantreibbar ist, wobei die Hilfseinrichtung ein den Endoskopschaft (1) umgebender Schlauch ist, dessen Grundflexibilität höher als die Grundflexibilität des Endoskopschafts (1) ist und **dadurch gekennzeichnet, dass** an dessen Außen- oder Innenumfang oder in dessen Wandung Hohlräume in Form von einer Mehrzahl von Fluidkissen (6) vorgesehen sind, die zur temporären Aussteifung des Schlauchs mit einem Druckmedium befüllbar sind, derart, dass die Schlauchflexibilität mittels der Druckerhöhung des in den jeweiligen Fluidkissen (6) sich befindlichen Mediums kleiner als die Grundflexibilität des Endoskopschafts (1) machbar ist und die Fluidkissen (6) dafür angepasst sind, sich durch den darin aufgebauten Druck zusätzlich gegen eine Wand des zu inspizierenden kanalartigen Hohlraums anzudrücken.

2. Endoskop nach Anspruch 1, **dadurch gekennzeichnet, dass** die Fluidkissen (6) in Umfangsrichtung verteilt sowie in in Axialrichtung überlappender Weise beabstandet sind.

3. Endoskop nach Anspruch 1 oder 2, **dadurch gekennzeichnet, dass** die Fluidkissen (6) zumindest an einem vorderen Endabschnitt des Schlauchs (5) angeordnet sind.

4. Endoskop nach Anspruch 3, **dadurch gekennzeichnet, dass** der vordere Endabschnitt, in welchem die Fluidkissen (6) angeordnet sind, ein Axiallänge von ca. 50cm beginnend vom distalen Schlauchende misst.

5. Endoskop nach einem der vorstehenden Ansprüche, **dadurch gekennzeichnet, dass** die Fluidkissen (6) so angeordnet sind, dass sie eine Anzahl von Gruppen formen, von denen jede Gruppe aus drei, in Umfangsrichtung beabstandeten Fluidkissen (6) besteht und die Gruppen in Axialrichtung des Schlauchs (5) beabstandet sind, wobei die Fluidkissen (6) zweier unmittelbar benachbarter Gruppen derart im Umfangsrichtung phasenverschoben sind, dass sie partiell zwischen sich zu liegen kommen und sich dabei in Axialrichtung überlappen.

## Claims

1. Endoscope having an alternating propulsion comprising an endoscope shaft (1) and an auxiliary means (5), wherein the endoscope is adapted to be propelled into a cavity by alternately retaining the endoscope shaft (1) and the auxiliary means (5) with respect to a canal-shaped cavity to be inspected and simultaneously propelling the element which is currently not being retained by guiding it along the element which is currently being retained, wherein the auxiliary means (5) is a hose which surrounds the endoscope shaft (1) and the basis flexibility of which is higher than the basis flexibility of the endoscope shaft (1), and **characterized in that** the hose (5) is provided with cavities at its inner or outer circumference or within its wall having the form a plurality of fluid pads (6) which can be filled with a medium for temporarily stiffening the hose (5), such that the flexibility of the hose (5) can be made smaller than the basis flexibility of the endoscope shaft (1) by increasing the pressure of the medium within the respective fluid pads (6), and wherein the fluid pads (6) are adapted to be additionally pressed against a wall of the canal-shaped cavity to be inspected by the pressure built-up within the fluid pads (6).

2. An endoscope according to claim 1, **characterized in that** the fluid pads (6) are distributed in the circumferential direction and are spaced apart in the axial direction in an overlapping manner.

3. An endoscope according to claim 1 or 2, **characterized in that** the fluid pads (6) are arranged at least at a leading end portion of the hose (5).

4. An endoscope according to claim 3, **characterized in that** the leading end portion, in which the fluid pads (6) are arranged, has an axial length of about 50cm starting from the distal end of the hose.

5. An endoscope according to one of the preceding claims, **characterized in that** the fluid pads (6) are arranged such that they form a number of groups, wherein each group consists of three fluid pads (6) spaced apart in the circumferential direction and the groups are spaced apart in the axial direction of the hose (5), wherein the fluid pads (6) of two directly neighboring groups are phase-shifted in the circumferential direction such that they lie partially between each other and thereby overlap in the axial direction.

## Revendications

1. Endoscope avec une propulsion alternative, avec une tige d'endoscope (1) et un dispositif auxiliaire (5), l'endoscope pouvant être entraîné en maintenant en alternance la tige de l'endoscope (1) et le dispositif auxiliaire (5) par rapport à un espace creux en forme de canal à inspirer et en avançant simultanément l'élément non maintenu droit avec un guidage le long de l'élément maintenu droit dans l'espace creux, le dispositif auxiliaire étant un tuyau entourant la tige de l'endoscope (1) et dont la flexibilité de base est supérieur à la flexibilité de base de la tige de l'endoscope (1) et **caractérisé en ce que**
au niveau de sa circonférence externe ou interne ou dans sa paroi, des espaces creux sous la forme d'une pluralité de coussins de fluide (6) sont prévus, qui peuvent être remplis pour une rigidification temporaire avec un fluide sous pression, de façon à ce que la flexibilité du tuyau puisse être rendue, au moyen de l'augmentation de la pression du fluide se trouvant dans le coussin de fluide (6) correspondant, inférieure à la flexibilité de base de la tige de l'endoscope (1) et les coussins de fluide (6) soient adaptés de façon à se comprimer, du fait de la pression régnant à l'intérieur, contre une paroi de l'espace creux en forme de canal à inspirer.

2. Endoscope selon la revendication 1, **caractérisé en ce que** les coussins de fluide (6) sont répartis sur la circonférence et éloignés de façon à se superposer dans la direction axiale.

3. Endoscope selon la revendication 1 ou 2, **caractérisé en ce que** les coussins de fluide (6) sont disposés au moins sur une portion d'extrémité avant du tuyau (5).

4. Endoscope selon la revendication 3, **caractérisé en ce que** la portion d'extrémité avant, dans laquelle les coussins de fluide (6) sont disposés, mesure une longueur axiale d'environ 50 cm à partir de l'extrémité distale du tuyau.

5. Endoscope selon l'une des revendications précédentes, **caractérisé en ce que** les coussins de fluide (6) sont disposés de façon à ce qu'ils forment une pluralité de groupes, chaque groupe étant constitué de trois coussins de fluide (6) répartis sur la circonférence et les groupes sont éloignés les uns des autres dans la direction axiale du tuyau (5), les coussins de fluide (6) de deux groupes immédiatement adjacents étant décalés sur la circonférence de façon à se poser les uns entre les autres et à se superposer dans la direction axiale.
